(19) Europäisches Patentamt
European Patent Office
Office européen des brevets

(11) **EP 3 769 032 B1**

(12) **EUROPÄISCHE PATENTSCHRIFT**

(45) Veröffentlichungstag und Bekanntmachung des Hinweises auf die Patenterteilung:
**05.01.2022   Patentblatt 2022/01**

(21) Anmeldenummer: **19703314.5**

(22) Anmeldetag: **05.02.2019**

(51) Int Cl.:
*G01B 9/02* (2006.01)          *G03H 1/04* (2006.01)
*G03H 1/06* (2006.01)          *G03H 1/08* (2006.01)
*A61B 3/10* (2006.01)

(86) Internationale Anmeldenummer:
**PCT/EP2019/052765**

(87) Internationale Veröffentlichungsnummer:
**WO 2019/179687 (26.09.2019 Gazette 2019/39)**

(54) **VERFAHREN ZUM ABLICHTEN EINER SEQUENZ VON SCHNITTFLÄCHEN IM INNERN EINES LICHT STREUENDEN OBJEKTS MIT VERBESSERTER ABTASTUNG**

METHOD FOR IMAGING A SEQUENCE OF CROSS SECTIONS INSIDE A LIGHT-SCATTERING OBJECT WITH IMPROVED SAMPLING

PROCÉDÉ DE CAPTURE D'IMAGES D'UNE SÉQUENCE DE SURFACES DE COUPE À L'INTÉRIEUR D'UN OBJET DIFFUSANT LA LUMIÈRE AVEC UN ÉCHANTILLONNAGE AMÉLIORÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.03.2018   DE 102018106292**

(43) Veröffentlichungstag der Anmeldung:
**27.01.2021   Patentblatt 2021/04**

(73) Patentinhaber: **Visotec GmbH**
**23552 Lübeck (DE)**

(72) Erfinder:
• **SUDKAMP, Helge**
**23552 Lübeck (DE)**
• **SPAHR, Hendrik**
**23562 Lübeck (DE)**
• **HILLMANN, Dierck**
**23552 Lübeck (DE)**
• **KOCH, Peter**
**23558 Lübeck (DE)**
• **HÜTTMANN, Gereon**
**23564 Lübeck (DE)**

(74) Vertreter: **Hermann, Felix**
**Boehmert & Boehmert**
**Anwaltspartnerschaft mbB**
**Pettenkoferstrasse 22**
**80336 München (DE)**

(56) Entgegenhaltungen:
EP-A1- 2 565 725          WO-A1-2010/092533
US-A1- 2016 252 880

• **PAVILLON N ET AL: "Iterative method for zero-order suppression in off-axis digital holography", OPTICS EXPRESS, OSA PUBLISHING, US, Bd. 18, Nr. 15, 19. Juli 2010 (2010-07-19) , Seiten 15318-15331, XP009155232, ISSN: 1094-4087, DOI: 10.1364/OE.18.015318 [gefunden am 2010-07-02]**
• **PAVILLON N ET AL: "Suppression of the zero-order term in off-axis digital holography through nonlinear filtering", APPLIED OPTICS, OPTICAL SOCIETY OF AMERICA, WASHINGTON, DC; US, Bd. 48, Nr. 34, 9. November 2009 (2009-11-09), Seiten H186-H195, XP001550272, ISSN: 0003-6935, DOI: 10.1364/AO.48.00H186**
• **HELGE SUDKAMP ET AL: "In-vivo retinal imaging with off-axis full-field time-domain optical coherence tomography", OPTICS LETTERS, Bd. 41, Nr. 21, 25. Oktober 2016 (2016-10-25), Seite 4987, XP055562783, US ISSN: 0146-9592, DOI: 10.1364/OL.41.004987**
• **HELGE SUDKAMP ET AL: "Simple approach for aberration-corrected OCT imaging of the human retina", OPTICS LETTERS, Bd. 43, Nr. 17, 27. August 2018 (2018-08-27), Seite 4224, XP055571705, US ISSN: 0146-9592, DOI: 10.1364/OL.43.004224**

EP 3 769 032 B1

**Beschreibung**

Die Erfindung betrifft ein Verfahren zum Ablichten von Schnittflächen im Innern eines Licht streuenden Objekts, wobei Licht einer kurzkohärenten Lichtquelle in einen Probenlichtstrahl und in einen Referenzlichtstrahl aufgeteilt und einem Probenarm und einem in seiner Länge veränderlichen Referenzarm zugeführt und hiernach auf einem zweidimensionalen Lichtdetektor überlagert wird.

Interferometrische Verfahren der gattungsgemäßen Art sind u.a. zur Optischen Kohärenztomographie (OCT) bekannt. Dabei ist es insbesondere möglich, mittels Freistrahl-Interferometern das gesamte von einer flächigen Probe gestreute Licht simultan zu detektieren ("full-field OCT, FF-OCT) und auf einem zweidimensionalen Lichtdetektor - z.B. eine CCD- oder CMOS-Kamera - mit dem Referenzlicht zu überlagern. Die so erzeugten und elektronisch erfassten zweidimensionalen Interferogramme enthalten u.a. Information über Schnittflächen im Probeninnern und können mittels Bearbeitung in einem Rechner ausgewertet werden, um diese Information zu extrahieren.

Die Druckschrift US 7,088,454 B2 schlägt beispielsweise ein solches FF-OCT-Verfahren vor, bei dem exemplarisch die in verschiedenen Tiefen einer Testprobe übereinander eingeprägten Schriftzüge aus den OCT-Messdaten herausgelesen werden. Die Lesetiefe, d.h. die Probentiefe, aus der gelesen wird, korrespondiert dabei mit der Länge des Referenzarmes. Für jede selektierte Lesetiefe werden zwei Interferogramme erfasst, die sich darin unterscheiden, dass das Referenzlicht einmal um $\pi/2$ phasenverschoben wird und einmal nicht. Zudem wird ein Hintergrundbild erfasst, indem der Referenzstrahl ausgeblendet wird. Die Schnittbilder werden aus diesen drei Bildern errechnet. Es wird auch festgestellt, dass Artefakte ("random noise") z.B. durch Vibrationen des Messaufbaus auftreten können. Zur Abhilfe schlägt die US 7,088,454 B2 vor, die Interferogramme mehrfach über ein Zeitintervall zu erfassen und hiernach die zeitlichen Mittelwerte zu berechnen und auszuwerten.

Aus der Druckschrift WO 2017/029160 A1 ist ein FF-OCT-Verfahren zu entnehmen, das einen verkippten Einfall des Referenzlichts auf den Lichtdetektor realisiert, wie man es ansonsten aus der Off-Axis Digitalen Holografie (OA-DH) kennt. Unter der Bedingung einer ausreichenden Größe der Speckles des Interferenzlichts auf der Kamera - im Verhältnis zum Pixelabstand P - werden verschiedene Phasenlagen des Referenzlichts simultan erfasst, und der Phasengradient entlang der Kamera dient zugleich der Trennung von Interferenz- und Hintergrundlicht mittels einer Fourier-Filterung.

EP 2 565 725 A1 offenbart ein Verfahren und eine Vorrichtung zum Herstellen eines digitalen Hologramms, das ein Objekt darstellt, umfassend das Erzeugen eines Messstrahls und zweier Referenzstrahlen, das Bestrahlen des Objekts mit dem Messstrahl und das Leiten des reflektierten Messstrahls zu einem optischen Sensor, das Leiten der Referenzstrahlen zu einer Spiegeleinrichtung, die sich unter einem Schrägwinkel erstreckt, und das Leiten der reflektierten Strahlen zu dem Sensor, um die Strahlen ein Interferenzmuster auf dem Sensor erzeugen zu lassen, Bereitstellen eines digitalen Signals, das das Muster auf dem Sensor repräsentiert, Verarbeiten des Signals, um ein digitales Hologramm zu erhalten, Unterziehen des digitalen Hologramms einer Fourier-Transformation im Ortsfrequenzbereich, um ein Spektrum zu erhalten, das einen DC-Term, zwei Bildterme ($R_1 *O$, $R_2 *O$) und zwei konjugierte Bildterme ($R_1 O*$, $R_2 O*$) umfasst, Filtern des Spektrums, um einen Term zu erhalten, der das Objekt repräsentiert, und Ersetzen eines Abschnitts eines Bildterms, der von dem DC-Term überlappt wird, durch den entsprechenden Abschnitt des anderen Bildterms.

WO 2010/092533 A1 offenbart ein Verfahren zur Vermessung der Topologie von Objekten mittels off-axis Digitalholographie, bei dem die auto- und kreuzkorrelierten Interferenzsignale im Fourierraum überlappfrei getrennt sind.

Pavillon et al., "Iterative method for zero-order suppression in off-axis digital holography", Optics Express, Vol. 18, Issue 15, pp. 15318-15331, 2010, schlägt eine Methode zur Unterdrückung des sogenannten Terms nullter Ordnung in einem Hologramm vor, die auf einem iterativen Prinzip basiert. Während der Hologrammaufnahme enthält die kodierte Information die Intensitäten der beiden Strahlen, die das Interferenzmuster erzeugen, die keine Informationen über die aufgezeichnete komplexe Wellenfront enthalten und das rekonstruierte Signal stören können. Die vorgeschlagene Methode unterdrückt selektiv den Term nullter Ordnung, indem sie die während der Wellenfront-Rekonstruktion gewonnenen Informationen in einem iterativen Verfahren verwendet und so seine Unterdrückung ohne jegliche a priori Kenntnisse über das Objekt ermöglicht. Die Methode wird analytisch analysiert und ihre Konvergenz wird untersucht. Dann wird ihre Leistungsfähigkeit experimentell gezeigt. Seine Robustheit wird durch die Anwendung des Verfahrens auf verschiedene Arten von Hologrammen, wie topographische Bilder von mikroskopischen Proben oder Speckle-Hologramme, beurteilt.

Pavillon et al., "Suppression of the zero-order term in off-axis digital holography through nonlinear filtering", Applied Optics, Vol. 48, Issue 34, pp. H186-H195, 2009, präsentiert die experimentelle Validierung einer neuen Rekonstruktionsmethode für die außeraxiale digitale holografische Mikroskopie (DHM). Diese Methode unterdrückt effektiv die Objekt-Autokorrelation, nämlich den Term nullter Ordnung, aus den holographischen Daten und verbessert dadurch die Rekonstruktionsbandbreite komplexer Wellenfronten. Der Algorithmus basiert auf nichtlinearer Filterung und kann auf Standard-DHM-Setups mit realistischen Aufnahmebedingungen angewendet werden. Wir untersuchen die Robustheit des Verfahrens unter verschiedenen experimentellen Konfigurationen und demonstrieren quantitativ seine Verbesserungsmöglichkeiten bei Phasensignalen.

Insbesondere verwendet das Verfahren der WO 2017/029160 A1 räumlich teilkohärentes Licht mit kurzer Kohärenzlänge - kleiner als 25 Mikrometer - aus dem NIR-Spektrum, und das Freistrahl-Interferometer weist einen durch einen verschiebbaren Referenzspiegel in seiner Länge veränderlichen Referenzarm auf. Zur Interferenz auf dem zweidimensionalen Lichtdetektor - z.B. einer CCD- oder CMOS-Kamera - am Ausgang des Interferometers kommt es nur bei Übereinstimmung der optischen Weglängen von Referenzlicht und gestreutem Probenlicht innerhalb der Kohärenzlänge. Der veränderliche Referenzarm erlaubt somit, Probenlicht aus verschiedenen Probentiefen mit dem Referenzlicht zur Interferenz zu bringen. Die Längenänderung des Referenzarmes kann durch den entlang der optischen Achse verschiebbaren Referenzspiegel nach Art der bekannten "time-domain" (TD) OCT realisiert werden, wobei eine repetierende Bewegung des Spiegels für wiederholte Tiefenscans möglich ist.

Das Verfahren der WO 2017/029160 A1 ist nach dem zuvor Gesagten durch den Begriff "Off-Axis Full-Field Time-Domain OCT" (OA-FF-TD-OCT) von anderen OCT-Verfahren klar abgegrenzt.

Wenn man mit einem der vorgenannten Verfahren diffus streuende Proben wie z.B. biologische Gewebe untersucht, dann spielt die Interferenz des Probenlichts mit sich selbst, d.h. die Überlagerung von in verschiedenen Tiefen gestreuten Lichtanteilen, eine Rolle.

Die halbe Bandbreite der Raumfrequenzen des kreuzkorrelierten Interferenzsignals - erzeugt durch die Überlagerung von Proben- und Referenzlicht auf der Kamera - wird durch die Zentralwellenlänge $\lambda_0$ und die numerische Apertur NA der Abbildung der Probe bestimmt und beträgt bei einer Abbildung mit Vergrößerungsfaktor M=1

$$\Delta k = \frac{2\,\pi\,NA}{\lambda_0}$$

Das autokorrelierte Interferenzsignal - durch Selbstinterferenz von Probenlicht - weist als halbe Bandbreite 2 $\Delta k$ auf, und beide Signale treten simultan in den elektronisch erfassten Interferogrammen auf. Dies bedeutet, die Fourier-Koeffizienten beider Signale sind nur wesentlich verschieden von null für Wellenzahlvektoren mit $|\vec{k}| < \Delta k$ für die Kreuzkorrelierten und $|\overline{k}| < 2\,\Delta k$ für die Autokorrelierten, d.h. sie treten bei der Fast Fourier Transform (FFT) der Interferogramme über einem gemeinsamen Definitionsbereich des Fourier-Raumes "vermischt" auf.

Durch die verkippte Einstrahlung des Referenzlichts wird ein Phasengradient entlang einer Achse in der Kameraebene eingerichtet, der zu einem detektierbaren Interferenzstreifenmuster (Fringes) führt. Diese periodische Modulation mit dem vorbestimmten Wellenzahlvektor $\overline{k}_F$ betrifft allein die Kreuzkorrelierten und verschiebt alle zugehörigen Fourier-Koeffizienten gegenüber denen der Autokorrelierten. Die Signale sind somit durch Fourier-Filterung trennbar, wenn man $|\overline{k}_F| \geq 3\,\Delta k$ einrichtet, weil die Definitionsbereiche der Fourier-Koeffizienten der beiden Signale dann sicher disjunkt sind.

Das beschriebene Vorgehen hat den Nachteil, dass man sich mit jeder realen Kamera mit endlich vielen lichtsensitiven Pixeln auch in einem endlichen Fourier-Raum bewegen muss, den man alles andere als günstig ausnutzt, wenn man die interessierenden Fourier-Koeffizienten des kreuzkorrelierten Signals in einen Randbereich dieses Fourier-Raums verschiebt. Typischerweise werden nicht einmal 10 % des verfügbaren Fourier-Raums für das Nutzsignal verwendet. Das theoretische Optimum liegt bei 50 %, da auch das komplex konjugierte Nutzsignal unvermeidlich erzeugt wird, das aber keine zusätzliche Information liefert.

Eine schlechte Ausnutzung des Fourier-Raums ist gleichbedeutend mit einer Überabtastung des Beitrages der Kreuzkorrelierten zu den Interferogrammen. Eine verbesserte Abtastung würde gestatten, ohne Erhöhung des Mess- oder Rechenaufwandes zu Schnittbildern der Probe mit erhöhter Auflösung und/oder vergrößertem Bildfeld zu gelangen.

Die Erfindung stellt sich daher die Aufgabe, das an sich bekannte OA-FF-TD-OCT-Verfahren dahingehend fortzubilden, dass eine verbesserte Abtastung des kreuzkorrelierten Interferenzsignals erreicht wird.

Die Aufgabe wird gelöst durch ein Verfahren zum Ablichten einer Sequenz von Schnittflächen im Innern eines Licht streuenden Objekts mit den Schritten:

- Bereitstellen einer Lichtquelle, die Licht mit einer vorbestimmten Zentralwellenlänge $\lambda_0$ und einer Kohärenzlänge kleiner als 25 Mikrometer emittiert;
- Aufteilen des Lichts der Lichtquelle in Proben- und Referenzlicht;
- flächiges Beleuchten des Objekts mit dem Probenlicht;
- Abbilden des vom Objekt gestreuten Probenlichts mit numerischer Apertur NA auf eine elektronische Kamera mit schachbrettartig mit Pixelabstand P angeordneten Pixeln;
- Interferieren lassen von Referenz- und Probenlicht auf der Kamera unter Einrichten eines Weglängenprofils und eines Phasengradienten des Referenzlichts entlang wenigstens einer vorbestimmten Achse in der Kameraebene;
- Verschieben des Weglängenprofils des Referenzstrahls mit einer zeitabhängigen Geschwindigkeit; und

- Erfassen weiterer Kamerabilder jeweils wenigstens indiziert mit einem Maß für die zeitabhängige Verschiebung des Weglängenprofils,

wobei die Bedingung $\frac{\sqrt{2}}{3+\sqrt{2}} \lambda_0 < \frac{2\,NA*P}{M} \leq \frac{\sqrt{2}}{1+\sqrt{2}} \lambda_0$ erfüllt wird. M ist der Vergrößerungfaktor der Abbildung.

Das erfindungsgemäße Verfahren umfasst ferner, dass für jedes indizierte Kamerabild wenigstens einer Teilmenge der erfassten Kamerabilder ein Umgebungsintervall aller Kamerabilder des Umgebungsintervalls errechnet wird und der errechnete Stapelmittelwert vom indizierten Kamerabild zur Errechnung eines strukturbereinigten Kamerabildes abgezogen wird.

Die abhängigen Ansprüche geben vorteilhafte Ausgestaltungen des Verfahrens an.

Es sei darauf hingewiesen, dass die vorliegende Erfindung eine Weiterbildung der Erfindung aus der WO 2017/029160 A1 darstellt und Letztere in vorteilhafter Weise ausgestaltet. Im Kontext dieser Beschreibung soll eine elektronische Kamera mit Pixeln im Abstand P (Mitte zu Mitte) verwendet werden. Der Begriff des Weglängenprofils wird aus der WO 2017/029160 A1 mit der folgenden Erläuterung zum Zustandekommen des kreuzkorrelierten Signals übernommen:

Sowohl die Speckle als auch die Fringes des Streifenmusters treten auf der Kamera nur in Erscheinung, wenn Referenz- und Probenlichtanteile auf den Kamerapixeln dieselbe Weglänge aufweisen und somit kohärent sind. Der Referenzstrahl definiert beim Einfall auf die Kamera ein Weglängenprofil auf den Pixeln entlang der Achse mit dem Phasengradienten. Die abgebildeten Objektpunkte, deren Streulicht dasselbe Weglängenprofil aufweist, liegen auf einer Schnittfläche im Innern des streuenden Objekts. In der Regel handelt es sich bei der Schnittfläche um eine Ebene, deren Normale gegen die optische Achse verkippt ist.

Das Weglängenprofil auf der Kamera hängt direkt von der Weglängenverteilung im Strahlquerschnitt des Referenzstrahls ab, wenn dieser auf die Kameraebene trifft. Wird das Referenzlicht über Spiegel oder Prismen abgelenkt, so dass es schräg einfällt, dann stimmen Phasenfronten und Pulsfronten des Lichts überein, d.h. im Strahlquerschnitt parallel zur Phasenfront ist die Weglänge überall dieselbe. Es stellt sich aber auf der Kamera ein lineares Weglängenprofil ein, das beispielsweise ein Weglängenintervall mit einer Intervallbreite von etwa 500 Wellenlängen $\lambda_0$ umfasst, also typisch einige Hundert Mikrometer. Streng genommen ist das Weglängenprofil auf der Kamera eine Funktion der beiden Pixelkoordinaten, aber es variiert hier nur entlang einer Achse, die von der Einfallsebene des Referenzlichts vorbestimmt ist.

Das Weglängenprofil des Referenzlichts auf der Kamera kann vom Nutzer vorgegeben werden. Dieses Profil legt fest, welche Schnittfläche des Objekts - hinsichtlich Orientierung, Form und Abstand zur Kamera - dann zu Interferenzen im erfassten Kamerabild beitragen kann. Insbesondere kann das Weglängenprofil durch Verändern der Referenzarmlänge verschoben, d.h. in seiner Gesamtheit zu kleineren oder größeren Werten überall auf der Kamera geändert werden. Dadurch werden weitere Schnittflächen des Objekts der Ablichtung zugänglich. Es kann eine Sequenz von Kamerabildern für die verschiedenen Schnittbilder während des Verschiebens des Weglängenprofils erfasst werden. Die Kamerabilder sind dabei wenigstens indiziert mit einem Maß für die Verschiebung des Weglängenprofils zu erfassen und gewöhnlich auch nicht-flüchtig abzuspeichern. Die Kamerabilder können überdies weitere Indizierungen tragen, beispielsweise durchnummeriert sein. Ein Maß für die Verschiebung kann ein Längenmaß, beispielsweise die Referenzarmlänge, sein oder auch ein Steuersignal für den Stellantrieb eines Referenzspiegels.

Weglängenprofil und Phasengradient sind nicht notwendig aneinander gekoppelt. Der Phasengradient hängt allein vom Einfallswinkel des Referenzstrahls auf die Kamera ab.

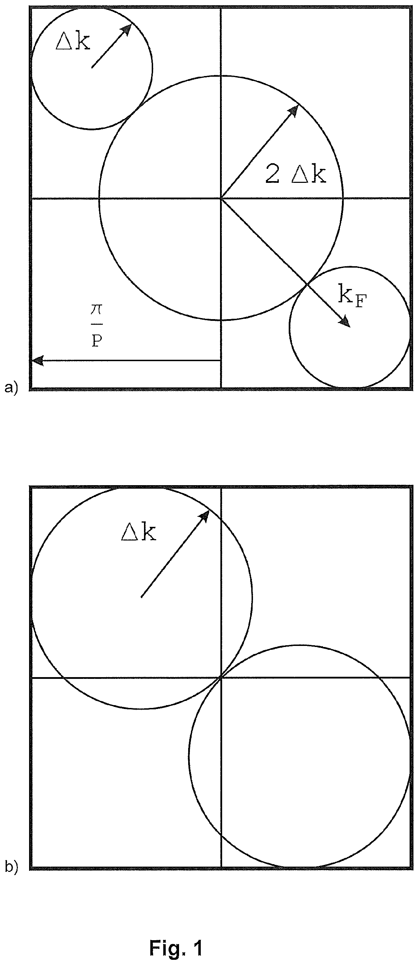

Zur nachfolgenden Erläuterung der Erfindung dient auch die einzige Fig. 1. Sie zeigt eine Skizze des auf eine quadratische Kamera bezogenen Fourier-Raumes mit den Definitionsbereichen der Fourier-Komponenten a) der kreuzkorrelierten und autokorrelierten Interferenzsignale nach dem Stand der Technik und b) allein des kreuzkorrelierten Nutzsignals gemäß der vorliegenden Erfindung.

Die WO 2017/029160 A1 sagt aus, dass die Abbildung des Objekts so einzurichten ist, dass der mittlere Speckle-Durchmesser D größer als zwei Pixelabstände P ist, und dass weiterhin der Phasengradient in der Kamera aus dem Intervall zwischen $2\pi/D$ und $\pi/P$ einzurichten ist. Dies ist äquivalent zur Forderung an den Einfallswinkel $\alpha$ des Referenzstrahls auf die Kameraebene:

$$\frac{NA}{1{,}22 * M} < \sin \alpha < \frac{\lambda_0}{2 * P}$$

Hierbei bezeichnet M den Vergrößerungsfaktor der Abbildung, d.h. das Verhältnis von Bildgröße zu Objektgröße, so dass $D = M*D_0$ gilt mit

$$D_0 = \frac{1,22 * \lambda_0}{NA}$$

als der Speckle-Größe bezogen auf die Objektebene. Wegen der zufälligen Verteilung der Lichtintensität in einem Specklefeld kann der Durchmesser der Speckle nur als statistische Größe angegeben werden, welche sich aus der Autokorrelationsfunktion berechnen lässt. Für eine kreisförmige Apertur ergibt sich eine Airy-Funktion (vgl. Dainty J., "The statistics of speckle patterns", Progress in Optics, E. Wolf, ed. (Elsevier), pp. 1-46, 1977). Gewöhnlich wird die erste Nullstelle als Maß für mittlere Speckle-Größe definiert, welche dann auch dem Durchmesser des Airy-Scheibchens der Punktspreizfunktion bei beugungsbegrenzter Auflösung entspricht.

Die rechte Seite der obigen Ungleichung verlangt die Abtastbarkeit des Streifenmusters mit der gegebenen Kamera (Nyquist-Bedingung), und die linke Seite der Ungleichung verlangt, dass das Streifenmuster in den einzelnen Speckles auf der Kamera erkennbar sein muss.

In Bezug auf die Fourier-Koeffizienten der Kreuzkorrelierten ist ein Streifenmuster mit einem Betrag des Wellenzahlvektors $k_F$ in der Nähe der Nyquist-Frequenz jedoch nicht zweckmäßig, weil dann feine Strukturen in den gesuchten Schnittflächen zu Frequenzen führen, die sich jenseits der Nyquist-Frequenz, d .h jenseits der Grenze der Abtastbarkeit, befinden würden.

Erfindungsgemäß ist es vielmehr von Vorteil, sich mit $|\overline{k}_F|$ eher der halben Nyquist-Frequenz anzunähern und den Betrag für die halbe Bandbreite $\Delta k$ des Nutzsignals durch Auswahl des mittleren Speckle-Durchmessers D günstig festzulegen.

Der Speckle-Durchmesser D wird durch zwei Bedingungen nach unten begrenzt:

(i) D muss mindestens so groß sein wie der Fringeabstand des durch den Phasengradienten auf der Kamera erzeugten Streifenmusters.

(ii) D muss so groß sein, dass die mit dem Phasengradienten modulierte Bandbreite des kreuzkorrelierten Nutzsignals keine Frequenzanteile jenseits der Nyquist-Frequenz der Kamera enthält.

Ist Bedingung (i) nicht erfüllt, dann kann man die Phasenlagen der Speckles nicht bestimmen, und verfehlt man Bedingung (ii), dann verliert man Nutzsignalinformation.

Für die halbe Bandbreite $\Delta k$ des Nutzsignals bedeutet Bedingung (ii) eine Obergrenze. Diese ist ersichtlich aus Fig. 1 a), in der der Fourier-Raum einer quadratischen Kamera skizziert ist. Die dargestellten Kreise beschreiben die Definitionsbereiche der von null verschiedenen Fourier-Koeffizienten des mit $k_F = |\overline{k}_F|$ modulierten kreuzkorrelierten Signals (links oben und rechts unten) und des autokorrelierten Signals (um k = 0), wobei die Kreisradien die halben Bandbreiten jeweils $\Delta k$ und 2 $\Delta k$ sind. Die Kreismittelpunkte sind dabei gerade um $|\overline{k}_F|$ = 3 $\Delta k$ gegeneinander verschoben. Damit die Definitionsbereiche einerseits nicht überlappen und andererseits vollständig im Fourier-Raum der Kamera liegen, muss $\Delta k \leq \frac{\sqrt{2}}{3+\sqrt{2}}\frac{\pi}{P}$ gewählt werden.

Wenn man das autokorrelierte Interferenzsignal nun aber gar nicht als ein Störsignal ansieht bzw. es einfach ignoriert, dann kann man, wie in Fig. 1 b) skizziert, eine Bandbreite bis hin zu $\Delta k = \frac{\sqrt{2}}{1+\sqrt{2}}\frac{\pi}{P}$ verwenden, bei der sich die Fourier-Komponenten nicht mit den komplex Konjugierten im Definitionsbereich (außer bei k=0) überlappen.

Erfindungsgemäß wird darauf abgezielt, das bislang für die Bildauswertung problematische Bandbreitenintervall

$$\frac{\sqrt{2}}{3+\sqrt{2}}\frac{\pi}{P} < \Delta k = 2\pi \frac{NA}{M * \lambda_0} \leq \frac{\sqrt{2}}{1+\sqrt{2}}\frac{\pi}{P}$$

zugänglich zu machen. Dies kann direkt in eine Anforderung an einen Messaufbau umformuliert werden, nämlich in

$$\frac{\sqrt{2}}{3+\sqrt{2}}\lambda_0 < \frac{2\,NA * P}{M} \leq \frac{\sqrt{2}}{1+\sqrt{2}}\lambda_0$$

als Einrichtungsvorschrift für die Kameraparameter in Beziehung zur benutzten Wellenlänge.

$$\left|\vec{k}_F\right| < 3\,\Delta k = 6\pi \frac{NA}{M*\lambda_0}$$

Den Phasengradienten kann man dann auch zu wählen. Um die halbe Bandbreite des Nutzsignals tatsächlich zu maximieren, sollte man vorzugsweise den Phasengradienten genau bei der halben Nyquist-Frequenz einrichten. In vielen praktischen Fällen strebt man aber keine Maximierung an, sondern nur eine Verbesserung. Dann kann man auch andere Phasengradienten, die immer noch kleiner sind als $|\vec{k}_F| = 3\,\Delta k$, durch eine andere Wahl des Einfallswinkels des Referenzlichts auf die Kamera einrichten.

Es ist anzumerken, dass in Fig. 1 die Nyquist-Frequenz $\frac{\sqrt{2}\pi}{P}$ entlang der Diagonalen beträgt, weil dort von einer quadratischen Kamera mit quadratischen, schachbrettartig angeordneten Pixeln und Pixelabstand P ausgegangen wird. Entlang der Diagonalen ist der effektive Pixelabstand dann um den Faktor $\sqrt{2}$ verkürzt, d.h. man hat eine höhere Abtastdichte, was an sich bekannt ist.

Der Speckle-Durchmesser auf der Kamera muss immer die beiden Bedingungen (i) und (ii) simultan erfüllen. An Bedingung (i) ändert sich durch die vorliegende Erfindung nichts. Durch die Verwendung kleinerer Phasengradienten als im Stand der Technik, erlaubt Bedingung (ii) nun erfindungsgemäß größere Bandbreiten und somit kleinere Speckle-Durchmesser als bisher. Die Speckle-Größe kann um einen Faktor bis hin zu:

$$\frac{\sqrt{2}+3}{\sqrt{2}+1} = 2\sqrt{2} - 1 \approx 1{,}82$$

verkleinert werden. Auch wenn man die Speckle-Größe D absichtlich etwas größer als nach den Bedingungen (i) und (ii) notwendig wählt, um z.B. einen Sicherheitsabstand zum Rand des Fourier-Raumes der Kamera zu haben, erzielt man bereits eine deutlich verbesserte Abtastung der Kreuzkorrelierten mit der gegebenen Kamera.

Mit der Erfindung lässt sich etwa der dreifache Fourier-Raum ausnutzen.

Es sind nun drei Sachverhalte festzuhalten und zu betonen:

I) Das Verfahren der WO 2017/029160 A1 wird durch die vorliegende Erfindung in einer Ausgestaltung präzisiert. Insbesondere kann man sich dadurch größere Freiheit bei der Wahl des Einfallswinkels des Referenzlichts auf die Kamera nehmen.

II) Das erfindungsgemäße Verfahren gewinnt mehr Strukturinformationen innerer Schnittflächen mit erhöhter Bildauflösung und/oder vergrößertem Bildfeld, weil man zu kleineren Speckle-Durchmessern bzw. zu größeren Bandbreiten des Nutzsignals übergehen kann als bislang im Stand der Technik möglich

III) Die tatsächliche Präsenz der Autokorrelierten in den erfassten Einzelbildern wird während der eigentlichen Bilderfassung schlichtweg ignoriert. Dabei werden keine isolierten Hintergrundbilder (ohne Referenz) erfasst, und die Autokorrelierten müssen auch nicht über alle Probentiefen gleich aussehen.

Es könnte den Fachmann zunächst überraschen, dass das Ignorieren der Autokorrelierten schadlos möglich sein soll.

Die Erfindung bezieht sich von vornherein auf eine Sequenz von Kamerabildern die unter Veränderung der Weglänge des Referenzlichts nach Art einer Time-Domain-OCT gewonnen wird. Kein einzelnes dieser Bilder ist einer unmittelbaren Filterung zur Trennung von Hintergrund und Nutzsignal zugänglich. Doch in der Zusammenschau der mit der Weglänge indizierten Bilder zeigt sich, dass eine erfindungsgemäße numerische Mittelungsprozedur das Nutzsignal in einem Mittelwertbild praktisch auslöscht. Es verbleibt dann ein immer noch stark strukturiertes Bild der Mittelwerte, das vom Referenzlicht unabhängig ist und insofern als Hintergrundbild aufgefasst werden kann. Dieses Hintergrundbild umfasst wesentlich die Autokorrelierten für die Probentiefe, aus deren Umgebung das Weglängenintervall zur Mittelung der indizierten Einzelbilder gewählt wird, und weiterhin auch alle statischen Strukturen aus dem apparativen Aufbau, z.B. Staubpartikel auf Linsen und dergleichen.

Es ist klar, dass man zu jedem mit der Weglänge $l_0$ des Referenzlichts indizierten und erfassten Einzelbild ein Weglängenintervall definieren kann. Dabei können zwei Fälle unterschieden werden:

Wenn sich die die Weglängen des Intervalls höchstens um die Kohärenzlänge $l_c$ des Lichts unterscheiden, z.B.

$\left[l_0 - \frac{l_c}{2}, l_0 + \frac{l_c}{2},\right]$ weisen die Einzelbilder zu Indizes aus diesem Intervall Speckles auf, die bereits bei Variation der Weglänge des Referenzlichts um $\lambda_0/2$ von konstruktiver zu destruktiver Interferenz wechseln. Verschiebt man den Referenzspiegel mit einem technischen Antrieb um mehrere Wellenlängen zwischen zwei Einzelbildern, dann ist die Helligkeit dieser Speckle praktisch eine um 0,5 verteilte Zufallsgröße. Addiert man nun mehrere solcher Bilder auf, so nehmen alle Speckle im Mittel denselben Wert 0,5 an, und die Struktur des Nutzsignals wird unsichtbar. Die Fourier-Koeffizienten der Kreuzkorrelierten werden - mit Ausnahme des Integrals, d.h. des Koeffizienten k=0 - zu null.

Für die Vermessung von größeren Probenvolumina kann es allerdings günstiger sein nur zwei oder weniger Messungen pro Kohärenzlänge durchzuführen, um die Messzeit zu reduzieren. Da die Messungen in dem Fall nicht mehr aus demselben Kohärenzvolumen stammen, sind die Speckle-Muster in den Kreuzkorrelierten bei Messungen in streuendem Gewebe in der Regel nicht mehr korreliert. Hier sind also die Phasen und auch die räumlichen Lagen der Speckle zufällig verteilt. Auch in diesem Fall können durch die Mittelung über eine nicht zu große Anzahl von Bildern - typisch bis zu 20 Bilder - die Kreuzkorrelierten ausgelöscht werden.

Ein Weglängenintervall der Form $[l_0, - \Delta l, l_0 + \Delta l,]$ soll als Umgebungsintervall der Weglänge $l_0$ bezeichnet werden. Dem Umgebungsintervall ist eine Teilmenge der erfassten Kamerabilder zugeordnet. Die Teilmenge kann auch als Stapel von Einzelbildern bezeichnet werden. Sind dies beispielsweise Bilder zu den Weglängen $l_j \in [l_0 - \Delta l, l_0 + \Delta l,]$, $j = 1,... ,N$ , so lautet ein gewichteter Stapelmittelwert

$$B_{av}(x, y, l_0) = \frac{1}{N} \sum_{j=1}^{N} \left[ B(x, y, l_j) - B_0(l_j) \right] \times G(l_j)$$

wobei $x,y$ Pixelkoordinaten bezeichnen und $B_{av}$, $B$, $B_0 \in [0,1]$ Helligkeitswerte (bzw. Graustufenwerte) auf den Pixeln sind. Die Parameter $B_0$ können dazu dienen, die mittlere Helligkeit aller Einzelbilder zu normieren, wenn dies erforderlich erscheint. Die Parameter G können als Gewichtsfaktoren wiederum bei Bedarf zur Normierung der Kontrastbreite der Bilder des Stapels dienen. Sie sind zudem geeignet, einzelne - z.B. offensichtlich fehlerhafte - Bilder aus der Betrachtung zu nehmen und/oder die effektive Reichweite der Summation zu begrenzen, z.B. durch abnehmende Gewichte mit dem Abstand zu $l_0$.

Letzten Endes wird der Anwender in Anbetracht einer erfassten Sequenz von Kamerabildern in einer Nachbearbeitung die genaue Wahl der Parameter der Stapelmittelung ermitteln. Die Ermittlung der Parameter ist dabei auch automatisierbar, insbesondere mit einer Software auf einem herkömmlichen Computer ausführbar, wenn dem Anwender die Eigenschaften der Bilderfassungseinrichtung bekannt sind und er konkrete Anforderungen an die Hintergrundbilder vorgibt. Ein beispielhaftes Kriterium dafür, dass der berechnete Stapelmittelwert eine gute Approximation für ein Hintergrundbild ohne Abhängigkeit von der Referenzarmlänge darstellt, ist seine langsame Veränderlichkeit aller Bildkomponenten beim Übergang von $l_0$ zu einer nahe gelegenen Tiefenebene, d.h. es sollte gelten $B_{av}(x,y,l_0) \approx B_{av}(x,y,l_0 + dl)$.

Nach der Bestimmung der Hintergrundbilder für alle erfassten Kamerabilder können im einfachsten und bevorzugten Fall die Differenzen

$$B'(x, y, l_0) = B(x, y, l_0) - B_{av}(x, y, l_0)$$

gebildet und die strukturbereinigten Bilder *B*' weiterverarbeitet werden. Es ist zweckmäßig, die strukturbereinigten Bilder dann einer Fourier-Transformation zu unterziehen und etwaig verbliebene Koeffizienten nahe k=0 sowie die komplex Konjugierten zu eliminieren (Fourier-Filterung). Die Fourier-Rücktransformation führt dann auf ein Abbild einer inneren Schnittfläche des abgelichteten Objekts indiziert mit der Weglängenverschiebung $l_0$ des Referenzlichts, d.h. die Schnittfläche kann einer Tiefenlage im Objekt zugewiesen werden.

Es ist also möglich, mit dem erfindungsgemäßen Ablichtungsverfahren für innere Schnittflächen streuender Objekte, das sich konkret auf eine Sequenz von Kamerabildern zu verschiedenen Weglängen des Referenzlichts bezieht und dabei die erfindungsgemäße Bedingung für die Abbildungsparameter beachtet, zu einem Datensatz zu gelangen, der in einer Nachbearbeitung Schnittbilder mit höherer Auflösung und/oder größerem Bildfeld als im Stand der Technik generiert. Diese Nachbearbeitung wird im Rahmen der Offenbarung der WO 2017/029160 A1 optional der Datenerfassung zeitlich nachgeschaltet, kann sogar erst sehr viel später durch einen Dritten erfolgen. Ebenso ist die Erzeugung des Rohdatensatzes selbst eine eigene werthaltige Leistung, deren Sinnhaftigkeit nach obigen Erläuterungen nicht in Zweifel steht.

Vorzugsweise dient die vorbeschriebene Erfindung der Ablichtung biologischer streuender Proben, besonders bevorzugt lebender Gewebe, ganz besonders bevorzugt der Ablichtung der Retina eines lebenden Auges. Neben den medizinischen Anwendungen in Ophthalmologie kann die Erfindung von einem Nutzer vorzugsweise auch verwendet werden zur biometrischen Verifikation der Identität des Nutzers.

**Patentansprüche**

1.  Verfahren zum Ablichten einer Sequenz von Schnittflächen im Innern eines Licht streuenden Objekts mit den Schritten:

    Bereitstellen einer Lichtquelle, die Licht mit einer vorbestimmten Zentralwellenlänge $\lambda_0$ und einer Kohärenzlänge kleiner als 25 Mikrometer emittiert;
    Aufteilen des Lichts der Lichtquelle in Proben- und Referenzlicht;
    flächiges Beleuchten des Objekts mit dem Probenlicht;
    Abbilden des vom Objekt gestreuten Probenlichts mit numerischer Apertur NA auf eine elektronische Kamera mit schachbrettartig mit Pixelabstand P angeordneten Pixeln;
    Interferieren lassen von Referenz- und Probenlicht auf der Kamera unter Einrichten eines Weglängenprofils und eines Phasengradienten des Referenzlichts entlang wenigstens einer vorbestimmten Achse in der Kameraebene;
    Verschieben des Weglängenprofils des Referenzstrahls mit einer zeitabhängigen Geschwindigkeit; und
    Erfassen weiterer Kamerabilder jeweils wenigstens indiziert mit einem Maß für die zeitabhängige Verschiebung des Weglängenprofils,
    wobei die Bedingung $\frac{\sqrt{2}}{3+\sqrt{2}}\lambda_0 < \frac{2\,NA*P}{M} \leq \frac{\sqrt{2}}{1+\sqrt{2}}\lambda_0$ erfüllt wird, wobei M der Vergrößerungfaktor der Abbildung ist;
    **gekennzeichnet dadurch, dass**
    für jedes indizierte Kamerabild wenigstens einer Teilmenge der erfassten Kamerabilder ein Umgebungsintervall der Indizierung vorbestimmt wird und ein gewichteter Stapelmittelwert aller Kamerabilder des Umgebungsintervalls errechnet wird und der errechnete Stapelmittelwert vom indizierten Kamerabild zur Errechnung eines strukturbereinigten Kamerabildes abgezogen wird.

2.  Verfahren nach Anspruch 1, **dadurch gekennzeichnet, dass** der Phasengradient entlang der vorbestimmten Achse in der Kameraebene auf die halbe Nyquist-Frequenz eingerichtet wird.

3.  Verfahren nach einem der vorangehenden Ansprüche, **gekennzeichnet durch** Errechnung der Abbilder der Schnittflächen im Innern des Objekts durch zweidimensionale Fourier-Filterung der strukturbereinigten Kamerabilder.

4.  Verfahren nach einem der vorangehenden Ansprüche, **dadurch gekennzeichnet, dass** das Licht streuende Objekt die Retina eines lebenden Auges ist.

5.  Verfahren nach Anspruch 4, **gekennzeichnet durch** biometrische Verifikation der Identität eines Nutzers.

**Claims**

1.  A method for illuminating a sequence of sectional areas in the interior of a light-scattering object with the steps:

    providing a light source, which emits light with a predetermined central wavelength $\lambda_0$ and a coherence length less than 25 microns;
    splitting the light of the light source into specimen light and reference light;
    flat illumination of the object with the specimen light;
    imaging of the specimen light scattered by the object with numerical aperture NA onto an electronic camera with pixels arranged in a checkerboard pattern with a pixel pitch P;
    causing interference of reference light and specimen light on the camera by establishing a path length profile and a phase gradient of the reference light along the predetermined axis in the camera plane;
    displacement of the path length profile of the reference beam at a time-dependent rate; and
    detection of further camera images in each case at least indexed with a dimension for the time-dependent displacement of the path length profile,
    wherein the condition $\frac{\sqrt{2}}{3+\sqrt{2}}\lambda_0 < \frac{2\,NA*P}{M} \leq \frac{\sqrt{2}}{1+\sqrt{2}}\lambda_0$ is met, wherein M denotes the magnification factor of the imaging;

**characterized in that**
for each indexed camera image of at least a part of the detected camera images, a surrounding interval of the indexing is predetermined and a weighted stack mean value of all the camera images of the surrounding interval is calculated and the calculated stack mean value of the indexed camera image is deducted from the indexed camera image for calculating a structurally improved camera image.

2. The method according to claim 1, characterized that the phase gradient along the predetermined axis in the camera plane is established on half the Nyquist frequency.

3. The method according to any one of the preceding claims, **characterized by** the calculation of the images of the sectional areas in the interior of the object by two-dimensional Fourier filtering of the structurally improved camera images.

4. The method according to any one of the preceding claims, **characterized in that** the light-scattering object is the retina of a living eye.

5. The method according to any one of claims 4, **characterized by** biometric verification of the identity of a user.


**Revendications**

1. Procédé d'illumination d'une séquence de surfaces de coupes à l'intérieur d'un objet diffusant la lumière, ayant les étapes suivantes :

   fourniture d'une source de lumière qui émet de la lumière ayant une longueur d'onde centrale $\lambda_o$ et une longueur de cohérence inférieure à 25 micromètres ;
   répartition de la lumière de la source de lumière en lumière d'échantillon et en lumière de référence ;
   éclairage à plat de l'objet avec la lumière de référence ;
   représentation de la lumière de référence diffusée par l'objet avec une ouverture numérique NA sur une caméra électronique avec des pixels disposés en damier avec une distance entre pixels P ;
   mise en interférence de la lumière de référence et de la lumière d'échantillon sur la caméra avec réglage d'un profil de longueur de trajet et d'un gradient de phase de la lumière de référence le long d'au moins un axe prédéfini dans le plan de la caméra ;
   déplacement du profil de longueur de trajet du faisceau de référence à une vitesse en fonction du temps ; et capture d'autres images de caméra respectivement au moins indexées avec une mesure du déplacement, en fonction du temps, du profil de longueur de trajet,

   la condition $\frac{\sqrt{2}}{3+\sqrt{2}} \lambda_0 < \frac{2\,NA*P}{M} \leq \frac{\sqrt{2}}{1+\sqrt{2}} \lambda_0$ étant remplie, M étant le facteur d'agrandissement de la représentation ;
   **caractérisé en ce que,**
   pour chaque image de caméra indexée d'au moins une quantité partielle des images de caméra capturées, un intervalle environnemental de l'indexation est prédéfini, et une valeur moyenne d'empilement pondérée de toutes les images de caméra de l'intervalle environnemental est calculée, et la valeur moyenne d'empilement calculée est extraite de l'image de caméra indexée pour le calcul d'une image de caméra corrigée des variations structurelles.

2. Procédé selon la revendication 1, **caractérisé en ce que** le gradient de phase le long de l'axe prédéfini dans le plan de caméra est réglé sur la demi-fréquence de Nyquist.

3. Procédé selon l'une des revendications précédentes, **caractérisé par** le calcul des images des surfaces de coupe à l'intérieur de l'objet par un filtrage de Fourier bidimensionnel des images de caméra corrigées des variations structurelles.

4. Procédé selon l'une des revendications précédentes, **caractérisé en ce que** l'objet diffusant la lumière est la rétine d'un œil vivant.

5. Procédé selon la revendication 4, **caractérisé par** une vérification biométrique de l'identité d'un utilisateur.

a)

b)

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7088454 B2
- WO 2017029160 A1
- EP 2565725 A1
- WO 2010092533 A1

**In der Beschreibung aufgeführte Nicht-Patentliteratur**

- **PAVILLON et al.** Iterative method for zero-order suppression in off-axis digital holography. *Optics Express,* 2010, vol. 18 (15), 15318-15331
- **PAVILLON et al.** Suppression of the zero-order term in off-axis digital holography through nonlinear filtering. *Applied Optics,* 2009, vol. 48 (34), H186-H195
- The statistics of speckle patterns. **DAINTY J.** Progress in Optics. Elsevier, 1977, 1-46